# FASCICULE DE BREVET EUROPEEN

(11) **EP 3 056 219 B1**
(45) Date de publication et mention de la délivrance du brevet: **22.08.2018**
(21) Numéro de dépôt: 16155443.1
(22) Date de dépôt: 12.02.2016
(51) Int. Cl.: A61K 47/36, A61K 9/00, A61K 36/185

(54) **COMPOSITION À BASE DE PLANTES DE LA FAMILLE DES MALVACEAE POUR UNE ADMINISTRATION SUR LES MUQUEUSES**
ZUSAMMENSETZUNG AUF PFLANZENBASIS DER FAMILIE DER MALVENGEWÄCHSE (MALVACEAE) ZUR VERABREICHUNG AUF SCHLEIMHÄUTEN
COMPOSITION BASED ON PLANTS OF THE MALVACEAE FAMILY FOR MUCOSAL DELIVERY

(30) Priorité: 12.02.2015 FR 1551143; 11.03.2015 FR 1552024
(43) Date de publication de la demande: 17.08.2016
(73) Titulaire: Laboratoires Chemineau, 37210 Vouvray (FR)
(72) Inventeur: DELAPLACE, Natacha, 37210 Chançay (FR); AGNUS, Benoit, 37110 Villedomer (FR)
(74) Mandataire: Cabinet Plasseraud

(56) Documents cités:
- WO-A1-99/27905
- DE-U1-202006 011 920
- US-A1- 2006 088 616
- ANONYMOUS: "Nasal Spray", GNPD; MINTEL, 1 juin 2012 (2012-06-01), XP002741530,
- ANONYMOUS: "Allergy Relief Nasal Spray", GNPD; MINTEL, 1 juin 2013 (2013-06-01), XP002741527,

## Description

La présente invention a pour objet une composition à base de plantes de la famille des Malvaceae, notamment de Mauve (Malva sylvestris, Althaea officinalis), telle que définie dans les revendications destinée à une administration sur les muqueuses.

L'invention a encore pour objet l'utilisation non-thérapeutique d'une telle composition pour protéger les muqueuses, notamment nasales, contre les agressions extérieures telles que les acariens, la pollution, les pollens etc. qui sont susceptibles notamment de causer des rhinites allergiques.

La rhinite allergique est une affection médicale bénigne secondaire à une hypersensibilisation à une substance étrangère dénommée allergène. Son expression clinique est essentiellement la rhinite et la conjonctivite. L'allergène peut être le pollen (dans le cadre du rhume des foins) mais aussi les acariens, ainsi que d'autres produits.

En France, pas moins d'une personne sur cinq est atteinte d'allergie et une fois sur deux, elle est respiratoire. Le nombre de victimes de rhinite allergique ne cesse d'augmenter. Cette affection trop négligée peut dégénérer vers une maladie beaucoup plus grave : l'asthme.

La rhinite allergique se caractérise par des éternuements à répétition, le «nez qui coule», des chatouillements dans le nez, des larmoiements, une irritation des yeux, et gâche donc ainsi véritablement la vie des personnes qui en souffrent. Il existe deux types de rhinites allergiques :
- la rhinite saisonnière ou rhume des foins, qui survient de manière intermittente, en réaction au pollen,
- la rhinite persistante (ou apériodique) qui est entretenue par des allergènes (acariens, pollution ...) constamment présents dans l'environnement de la personne.

Les traitements contre les rhinites sont souvent des antihistaminiques, des décongestionnants pour le nez, des gouttes pour les yeux. Certaines fois, si les symptômes sont vraiment très gênants, des corticoïdes peuvent également être prescrits.

Une désensibilisation est également possible en guise de traitement pour réduire les manifestations allergiques, voire les faire disparaitre. La désensibilisation consiste à administrer un allergène de façon continue sur plusieurs mois ou années. Administrée par voie sous-cutanée, la désensibilisation a prouvé son efficacité, mais au prix parfois d'effets indésirables (réactions locales aux points d'injections, urticaire), parfois graves (bronchospasme, angio-oedème, asthme, réaction anaphylactique).

Concernant la pollution, de nombreuses études permettent aujourd'hui d'affirmer que même à des niveaux faibles, cette dernière a des effets néfastes sur notre santé.

Des compositions à base de plantes de la famille des Malvaceae, et notamment à base de Mauve, pour prévenir et/ou traiter les rhinites, ainsi que pour leur action anti-allergique sont connues de l'art antérieur.

Ainsi, le document DE 20 2006 011920 décrit des compositions pour la prévention et/ou le traitement topique de rhinites, ladite composition comprenant un mucilage et du chlorure de sodium. Un exemple de mucilage cité est notamment la mauve.

Le document US 2006/0088616 décrit une composition comprenant un extrait de Malva sylvestris L. pour stimuler la production du mucus, et protéger les muqueuses, et notamment la muqueuse nasale.

Cependant, il subsiste toujours à ce jour la nécessité de trouver de nouvelles compositions qui soient particulièrement bien adaptées pour une application au niveau des muqueuses et notamment de la muqueuse nasale.

Un des buts de la présente invention est de trouver une nouvelle composition à base d'une plante de la famille des Malvaceae, qui forme un film uniforme lorsqu'elle est appliquée au niveau des muqueuses, notamment de la muqueuse nasale, et qui présente une viscosité satisfaisante pour les muqueuses.

Un autre but de l'invention est de trouver une composition à base d'une plante de la famille des Malvaceae, qui lorsqu'elle est appliquée au niveau des muqueuses, notamment de la muqueuse nasale, ne soit pas irritante pour les muqueuses.

Les Inventeurs ont ainsi imaginé une nouvelle composition, qui lorsqu'elle est administrée au niveau de la muqueuse, protège efficacement ladite muqueuse de son environnement immédiat par création d'un film protecteur, hydratant et qui présente une viscosité satisfaisante et qui en outre ne soit pas irritante pour la muqueuse. En particulier, lorsque cette composition est appliquée sur la muqueuse nasale, elle présente en outre l'avantage d'assurer le bon maintien de la motilité ciliaire.

La présente invention a ainsi pour objet une composition filmogène caractérisée en ce qu'elle comprend :
- un extrait de plantes de la famille des Malvaceae, de préférence du genre Althaea ou Malva,
- un agent filmogène, choisi dans le groupe comprenant un polysaccharide anionique ayant pour nom INCI biosaccharide gum-1, du xylitol, de l'acacia, du collagène, de l'acide hyaluronique et des mucopolysaccharides, et
- un émulsifiant non ionique, de préférence présentant des propriétés viscosifiantes, choisi dans le groupe comprenant un poloxamer, de la gomme xanthane, de la gomme de sclérotium, de l'agar agar, et de préférence un poloxamer,
   ladite composition étant exempte de chlorure de sodium.
Selon l'invention, cette composition est avantageusement destinée à une administration au niveau de la muqueuse nasale. Ainsi, cette composition pourra être pulvérisée dans la cavité nasale au moyen d'une pompe distributrice ou d'une valve.

Selon l'invention, l'extrait de plantes de la famille des Malvaceae est par exemple un extrait de Mauve, notamment choisi parmi Malva sylvestris ou Althaea officinalis.

Selon un mode de réalisation de l'invention, l'extrait de Mauve (Malva sylvestris ou Althae officinalis) sera plus particulièrement un extrait aqueux ou un extrait hydroalcoolique. A titre d'exemple d'un solvant d'extraction hydroalcoolique on pourra citer un mélange eau/butylèneglycol dans les proportions 50/50.

Selon un autre mode de réalisation, l'extrait de mauve (Malva sylvestris ou Althae officinalis) sera un extrait sec.

Parmi les plantes ornementales et utiles de la famille des Malvaceae, outre les genres Malva et Althaea, on pourra également citer les genres Alcea, Gossypium et Hibiscus.

L'extrait peut provenir de la fleur mais également de toute autre partie de la plante, telle que les racines, la tige, les feuilles.

Pour Malva sylvestris, l'extrait est un extrait de fleurs.

L'utilisation d'un extrait de plantes de la famille des Malvaceae, notamment de Mauve, permet avantageusement de ne pas bloquer la motilité ciliaire naturellement présentée par les cils de la muqueuse nasale, permet également de favoriser la clearance mucociliaire et d'hydrater par sa composition riche en mucillage.

On entend par « motilité ciliaire » le mouvement vibratoire naturellement effectué par les cils de la muqueuse nasale. Ces cils contribuent à éliminer la poussière et les agents infectieux de l'air inspiré, avant qu'il n'atteigne l'appareil respiratoire inférieur. Ces cils jouent également un rôle important dans l'olfaction : lorsqu'une substance odorante active les cils, les récepteurs répondent en envoyant une série d'influx nerveux à analyser au cerveau.

On entend par « clearance mucociliaire », l'action combinée du mucus et des cils de la muqueuse respiratoire pour éliminer les particules étrangères.

L'extrait de plantes de la famille des Malvaceae, notamment de fleurs de Mauve, est également avantageusement utilisé pour ses propriétés émollientes.

La composition selon l'invention comprend un agent filmogène de façon à ce que, lors de l'application, la composition forme un film, c'est-à-dire une fine couche continue.

Selon un mode de réalisation particulier, l'agent filmogène est un polysaccharide neutre ou anionique. A titre d'exemple le polysaccharide anionique est le biosaccharide gum-1. Ce polysaccharide anionique présente l'avantage supplémentaire d'être hydratant et ainsi il est tout à fait approprié à une application sur les muqueuses nasales.

Selon un autre mode de réalisation particulier, l'agent filmogène est de l'acide hyaluronique.

La composition selon l'invention comprend également un émulsifiant non ionique. Cet émulsifiant non ionique peut présenter des propriétés viscosifiantes. Cet émulsifiant peut être tel que défini ci-dessus, et est notamment un polymère. A titre d'exemple de tel émulsifiant non ionique présentant de préférence des propriétés viscosifiantes, on peut citer les poloxamers, et notamment le poloxamer 188 ou le poloxamer 407.

Un autre émulsifiant non ionique pouvant être cité selon l'invention est la gomme xanthane.

Les Inventeurs ont découvert de manière tout à fait surprenante que l'association d'un extrait de Mauve, notamment Malva sylvestris ou Althaea officinalis, avec un émulsifiant non ionique, de préférence présentant des propriétés viscosifiantes, et notamment un poloxamer, permettait d'augmenter de façon inattendue la viscosité de la composition de l'invention.

Il est connu de l'état de l'art que la présence d'extraits de plantes, et notamment d'extraits aqueux de plantes, contribue à la diminution de la viscosité de compositions comprenant des agents viscosants et/ou des agents gélifiants. La raison invoquée est la concentration en électrolytes dans les extraits aqueux de plantes.

Les compositions de l'invention présentent ainsi la particularité de présenter, contre toute attente, une viscosité augmentée lors de l'association « extrait de Mauve » avec un émulsifiant non ionique, de préférence présentant des propriétés viscosifiantes, et notamment un poloxamer.

On entend par « viscosité améliorée », « viscosité augmentée » ou « viscosité satisfaisante pour la muqueuse nasale », au sens de la présente invention, une viscosité dynamique allant de 3,5 mPa.s à 40 mPa.s lorsque la viscosité de la composition de l'invention est mesurée à une température de 25°C avec un viscosimètre Rhéomat RM 200 dans les conditions suivantes :
- système de mesure 1.1 (godet 1 et mobile 1) ;
- précisaillement 7 secondes à 500s⁻¹ ;
- cisaillement 15 secondes à 500 s⁻¹ ;
- 8 points de mesure.

Les compositions de l'invention présentent également l'avantage d'être hypotoniques. On entend par composition « hypotonique » au sens de la présente invention une composition présentant une osmolalité inférieure ou égale à 300 mOsml/kg (milliosmoles par kilogramme) lorsque l'osmolalité de la composition est mesurée à l'aide d'un osmomètre cryoscopique.

Pour rappel, la référence de l'osmolalité est le plasma sanguin qui présente une osmolalité allant de 280 à 300 mOsml/kg.

L'osmolalité des compositions de l'invention leur confère notamment les avantages de ne pas être irritantes pour les muqueuses, notamment la muqueuse nasale.

Selon un autre mode de réalisation de l'invention, la composition telle que définie ci-dessus comprend en outre :
- un agent conservateur, de préférence choisi dans le groupe comprenant le sorbate de potassium, l'acide sorbique, l'éthylhexylglycérine, le pentylèneglycol, l'acide lactique et ses sels, et leurs mélanges,
- un agent ajusteur de pH, de préférence choisi dans le groupe comprenant l'acide citrique, l'acide acétique, l'acide chlorhydrique, et leurs mélanges,
- un solvant, de préférence choisi dans le groupe comprenant l'eau purifiée, l'eau osmosée, l'eau pour préparation injectable PPI, l'eau de mer naturelle ou reconstituée, l'eau permutée, le sérum physiologique, et leurs mélanges,
- éventuellement un actif, de préférence issu d'une plante habituellement utilisée pour la sphère ORL.

A titre d'exemple de plante dont les actifs sont habituellement utilisés pour la sphère ORL, on peut citer l'eucalyptus, le thym, la marjolaine, l'origan, la camomille, le clou de girofle, la cannelle, le ravintsara, et leurs mélanges.

Selon un mode de réalisation particulier, dans la composition telle que définie ci-dessus :
- l'agent conservateur est le sorbate de potassium,
- l'agent ajusteur de pH est l'acide citrique,
- le solvant est l'eau purifiée,
- l'actif est une plante choisie dans le groupe comprenant l'eucalyptus, le thym, la marjolaine, l'origan, la camomille, le clou de girofle, la cannelle, le ravintsara, et leurs mélanges.

Selon un mode de réalisation particulier, la composition selon l'invention est exempte de chlorure de sodium.

L'absence de chlorure de sodium permet notamment à la composition de l'invention d'être hypotonique. Lors de l'application de la composition au niveau de la muqueuse nasale, on évite ainsi tout risque d'irritabilité.

Selon un autre mode de réalisation particulier, la composition selon l'invention est exempte de saponine triterpénique.

Selon un mode de réalisation de l'invention, dans la composition telle que définie ci-dessus:
- l'extrait de plantes, et notamment de Mauve, est présent en une quantité en poids allant de 0,02% à 10%, de préférence de 0,05% à 1,5% en poids, et plus préférentiellement encore est présent en une quantité de 0,1% à 0,2%,
- l'agent filmogène, et notamment le polysaccharide anionique, est présent en une quantité en poids allant de 0,02% à 5,0%, de préférence de 0,04% à 0,8%, et plus préférentiellement encore est présent en une quantité de 0,06%,
- l'émulsifiant non ionique, de préférence présentant des propriétés viscosifiantes, est présent en une quantité en poids allant de 0,01% à 15%, de préférence de 0,3% à 11%, et plus préférentiellement encore de 1% à 8%,
- l'agent conservateur est présent en une quantité allant de 0,07% à 0,5%, de préférence de 0,09% à 0,2%, et plus préférentiellement encore est présent en une quantité de 0,125% en poids,
- l'agent ajusteur de pH est ajouté en une quantité permettant un ajustement de pH allant de 2 à 8, de préférence de 3 à 7,5, et plus préférentiellement encore de 3,5 à 6,5,
- le solvant est ajouté en une quantité qsp 100%,
chacune desdites quantités en poids étant définie par rapport au poids total de la composition.

La présence combinée de :
- l'extrait de Mauve, notamment choisi parmi Malva sylvestris ou Althaea officinalis,
- l'émulsifiant à propriétés viscosifiantes, et notamment le poloxamer, et de
- l'agent filmogène, et notamment un polysaccharide neutre ou anionique,
dans les compositions de l'invention favorise la formation et la tenue d'un film homogène, présentant une viscosité satisfaisante et ne présentant aucun risque d'irritation pour la muqueuse sur laquelle il se forme. En outre, lorsque les compositions de l'invention sont appliquées sur la muqueuse nasale, le film formé permet le maintien de la motilité ciliaire naturellement présentée par les cils de la muqueuse nasale.

Ainsi selon l'invention, lorsque la composition est déposée sur les muqueuses, et en particulier dans la cavité nasale, elle forme au contact des muqueuses un film aux propriétés avantageuses ci-dessus décrites.

L'invention a également pour objet une composition telle que définie ci-dessus, caractérisée en ce qu'elle comprend une viscosité dynamique allant de 3,5 mPa.s à 40 mPa.s lorsque la viscosité de la composition de l'invention est mesurée à une température de 25°C avec un viscosimètre Rhéomat RM 200 dans les conditions suivantes :
- système de mesure 1.1 (godet 1 et mobile 1) ;
- précisaillement 7 secondes à 500s⁻¹ ;
- cisaillement 15 secondes à 500 s⁻¹ ;
- 8 points de mesure.

L'invention a encore pour objet une composition telle que définie ci-dessus, caractérisée en ce qu'elle comprend une osmolalité inférieure ou égale à 300 mOsml/kg lorsque l'osmolalité de la composition est mesurée à l'aide d'un osmomètre cryoscopique

Un autre objet de l'invention réside encore dans un dispositif de pulvérisation nasale comprenant une composition telle que définie ci-dessus. La composition de l'invention se présente avantageusement sous la forme d'un liquide dans le dispositif de pulvérisation.

Un tel dispositif de pulvérisation permet avantageusement l'administration de la composition dans la cavité nasale en doses.

A titre d'exemple de doses on pourra citer des doses allant de 30 microlitres à 300 microlitres de composition pour une pulvérisation unique et dans une cavité nasale.

Selon l'invention, le dispositif de pulvérisation nasale pourra se présenter sous forme de flacon pompe, de flacon pressurisé en aérosol, de flacon pompe airless ou de valves à poches, et se présentera de préférence sous forme de flacon pompe.

L'invention a encore pour objet l'utilisation d'une composition telle que définie ci-dessus, pour protéger les muqueuses nasales contre les agressions extérieures telles que les acariens, la pollution ou les pollens.

L'invention a également pour objet une composition telle que définie ci-dessus pour une utilisation dans la prévention et/ou le traitement des symptômes de la rhinite allergique, notamment causée par les agressions extérieures telles que pollens, acariens et/ou pollution.

Lorsque la composition de l'invention est utilisée pour prévenir et/ou traiter les symptômes de la rhinite allergique, et/ou pour protéger les muqueuses nasales des agressions extérieures, alors la composition est administrée par pulvérisation dans chaque cavité nasale, deux ou trois fois par jour, et à raison d'une ou deux pulvérisations dans chaque cavité nasale.

Les exemples suivants illustrent l'invention, ils ne la limitent en aucune façon.
La figure 1 illustre la viscosité des compositions 1 à 3 de l'invention comparées à un témoin (composition 11) en fonction de la température.
La figure 2 illustre le temps d'écoulement des compositions 1 à 3 de l'invention comparées à un témoin (composition 11) en fonction de la température.
La figure 3 est un test de fluorescéine permettant d'observer l'uniformité du film formé par la composition 1 de l'invention (voir figure 3a). La figure 3b est un témoin.

### Exemple 1 : compositions de spray nasal de l'invention à base de Malva sylvestris et de poloxamer 188

Des compositions de spray nasal selon l'invention sont respectivement données dans les tableaux 1 et 2 ci-dessous.

**Tableau 1**

| Composition **1** | **% (m/m) incorporation dans la composition 1** |
|---|---|
| **Dénomination INCI (« International Nomenclature of Cosmetic Ingredient »)** | |
| Extrait de fleurs de Mauve (*Malva sylvestris*) à 10-20% | 1% |
| Biosaccharide gum-1 à 1% (commercialisé sous la dénomination Fucogel® 1,5P) | 6% |
| Poloxamer 188 (commercialisé sous la dénomination Lutrol® F68) | 5% |
| Sorbate de potassium | 0,125% |
| Acide citrique | Qsp pH=5 |
| Eau purifiée | Qsp 100% |

6% de biosaccharide gum-1 à 1% correspond à une quantité en poids de 0,06% par rapport au poids total de la composition.

**Tableau 2**

| Composition **2** | **% (m/m) incorporation dans la composition 2** |
|---|---|
| **Dénomination INCI (« International Nomenclature of Cosmetic Ingredient »)** | |
| Extrait de fleurs de Mauve (*Malva sylvestris*) à 10-20% | 1% |
| Biosaccharide gum-1 (Fucogel® 1,5P) à 1% | 6% |
| Poloxamer 188 (Lutrol® F68) | 10% |
| Sorbate de potassium | 0,125% |
| Acide citrique | Qsp pH=5 |
| Eau purifiée | Qsp 100% |

L'extrait de Malva sylvestris (N° CAS 84082-57-5) provient de la société Herbarom Laboratoire. Cet extrait correspond à un extrait hydroalcoolique (eau/butylène glycol dans les proportions 50v/50v) de fleurs de mauve.

Le Biosaccharide gum-1 est utilisé pour ses propriétés filmogènes et hydratantes.

### Exemple 2 : compositions de spray nasal de l'invention à base de Malva sylvestris et de poloxamer 407

Des compositions de spray nasal selon l'invention sont respectivement données dans les tableaux 3 et 4 ci-dessous.

**Tableau 3**

| Composition **3** | **% (m/m) incorporation dans la composition 3** |
|---|---|
| **Dénomination INCI (« International Nomenclature of Cosmetic Ingredient »)** | |
| Extrait de fleurs de Mauve (*Malva sylvestris*) à 10-20% | 1% |
| Biosaccharide gum-1 (Fucogel® 1,5P) à 1% | 6 |
| Poloxamer 407 (commercialisé sous la dénomination Lutrol ® F127) | 5% |
| Sorbate de potassium | 0,125% |
| Acide citrique | Qsp pH=5 |
| Eau purifiée | Qsp 100% |

**Tableau 4**

| Composition **4** | **% (m/m) incorporation dans la composition 4** |
|---|---|
| **Dénomination INCI (« International Nomenclature of Cosmetic Ingredient »)** | |
| Extrait de fleurs de Mauve (*Malva sylvestris*) à 10-20% | 1% |
| Biosaccharide gum-1 à 1% (commercialisé sous la dénomination Fucogel® 1,5P) | 6% |
| Poloxamer 407 (Lutrol ® F127) | 10% |
| Sorbate de potassium | 0,125% |
| Acide citrique | Qsp pH=5 |
| Eau purifiée | Qsp 100% |

### Exemple 3 : compositions de spray nasal de l'invention à base d'Althea officinalis et de poloxamer 188

Des compositions de spray nasal selon l'invention sont respectivement données dans les tableaux 5 et 6 ci-dessous.

Le numéro CAS de l'extrait d'*Althea officinalis* est le 73049-65-7.

**Tableau 5**

| Composition **5** | **% (m/m) incorporation dans la composition 5** |
|---|---|
| **Dénomination INCI (« International Nomenclature of Cosmetic Ingredient »)** | |
| Extrait de Mauve (*Althea officinalis*) à 1-5% | 2% |
| Biosaccharide gum-1 à 1% (commercialisé sous la dénomination Fucogel® 1,5P) | 6% |
| Poloxamer 188 (Lutrol® F68) | 5% |
| Ethylhexylglycérine | 0,125% |
| Acide citrique | Qsp pH=5 |
| Eau purifiée | Qsp 100% |

**Tableau 6**

| Composition **6** | **% (m/m) incorporation dans la composition 6** |
|---|---|
| **Dénomination INCI (« International Nomenclature of Cosmetic Ingredient »)** | |
| Extrait de Mauve (*Althea officinalis*) à 1-5% | 2% |
| Biosaccharide gum-1 à 1% (commercialisé sous la dénomination Fucogel® 1,5P) | 6% |
| Poloxamer 188 (Lutrol® F68) | 10% |
| Ethylhexylglycérine | 0,125% |
| Acide citrique | Qsp pH=5 |
| Eau purifiée | Qsp 100% |

### Exemple 4 : compositions de spray nasal de l'invention

### à base d'Althea officinalis et de poloxamer 407

Des compositions de spray nasal selon l'invention sont respectivement données dans les tableaux 7 et 8 ci-dessous.

**Tableau 7**

| Composition **7** | **% (m/m) incorporation dans la composition 7** |
|---|---|
| **Dénomination INCI (« International Nomenclature of Cosmetic Ingredient »)** | |
| Extrait de Mauve (*Althea officinalis*) à 1-5% | 2% |
| Biosaccharide gum-1 à 1% (commercialisé sous la dénomination Fucogel® 1,5P) | 6% |
| Poloxamer 407 (Lutrol® F127) | 5% |
| Ethylhexylglycérine | 0,125% |
| Acide citrique | Qsp pH=5 |
| Eau purifiée | Qsp 100% |

**Tableau 8**

| Composition **8** | **% (m/m) incorporation dans la composition 8** |
|---|---|
| **Dénomination INCI (« International Nomenclature of Cosmetic Ingredient »)** | |
| Extrait de Mauve (*Althea officinalis*) à 1-5% | 2% |
| Biosaccharide gum-1 à 1% (commercialisé sous la dénomination Fucogel® 1,5P) | 6% |
| Poloxamer 407 (Lutrol® F127) | 10% |
| Ethylhexylglycérine | 0,125% |
| Acide citrique | Qsp pH=5 |
| Eau purifiée | Qsp 100% |

### Exemple 5 : compositions de spray nasal de l'invention à base d'Althea officinalis, d'acide hyaluronique et de gomme xanthane

Des compositions de spray nasal selon l'invention sont respectivement données dans les tableaux 9 et 10 ci-dessous.

**Tableau 9**

| Composition **9** | **% (m/m) incorporation dans la composition 9** |
|---|---|
| **Dénomination INCI (« International Nomenclature of Cosmetic Ingredient »)** | |
| Extrait de Mauve (*Althea officinalis*) à 10-20% | 1% |
| Acide hyaluronique (commercialisé sous dénomination cristahyal 2.2) | 0,15% |
| Gomme xanthane à 55-45% (commercialisée sous la dénomination Thixogum) | 0,05% |
| Ethylhexylglycérine | 0,125% |
| Acide citrique | Qsp pH=5 |
| Eau purifiée | Qsp 100% |

0,05% de gomme xanthane à 55%-45% correspond à une quantité en poids de 0,0275%-0,0225% par rapport au poids totale de la composition.

**Tableau 10**

| Composition **10** | **% (m/m) incorporation dans la compo. 10** |
|---|---|
| **Dénomination INCI (« International Nomenclature of Cosmetic Ingredient »)** | |
| Extrait de Mauve (*Althea officinalis*) à 10-20% | 1% |
| Acide hyaluronique (commercialisé sous dénomination cristahyal 2.2) | 0,10% |
| Gum Xanthane à 55-45% (commercialisé sous la dénomination Thixogum) | 0,1% |
| Ethylhexylglycérine | 0,125% |
| Acide citrique | Qsp pH=5 |
| Eau purifiée | Qsp 100% |

### Exemple 6: stabilité des compositions de l'invention

Des essais de stabilité des compositions selon les exemples 1 à 5 ont été conduits.

Toutes les compositions 1 à 10 se sont avérées stables dans les conditions testées, à savoir 24 mois à 25°C / 60% d'humidité résiduelle(HR).

### Exemple 7 : Impact des principaux constituants de la composition de l'invention sur la viscosité

Le but de cet exemple est d'étudier l'impact sur la viscosité des composants principaux de la composition filmogène de l'invention.

Différents essais ont été effectués avec le viscosimètre Rhéomat RM 200 dans les conditions suivantes :
- système de mesure 1.1 (godet 1 et mobile 1) ;
- précisaillement : 7 secondes à 500s⁻¹ ;
- cisaillement 15 secondes à 500 s⁻¹ ;
- 8 points de mesure ;
- température de 25°C.

Les solutions analysées sont dénommées A à I, et la composition de chacune de ces solutions est indiquée dans le tableau 11. La viscosité de chacune des solutions est indiquée dans le tableau 12.

La solution de base de travail dans cet exemple est le mélange D constitué d'eau et de butylèneglycol dans les proportions 50v/50v.

L'extrait de plantes de la famille des Malvaceae est un extrait eau/butylène glycol dans les proportions 50v/50v de fleurs de mauve et correspond plus particulièrement à la solution G.

**Tableau 11**

| **Solution** | **Préparation des solutions** | **Résumé de la composition de la solution** | | | |
|---|---|---|---|---|---|
| A | Eau | Eau | / | / | / |
| B | Ajout de fucogel à hauteur de 10% dans eau | Eau | / | 10 % Fucogel | / |
| C | Ajout de lutrol à hauteur de 10% dans eau | Eau | / | 10 % Lutrol | / |
| D | Mélange 50% eau/ 50% Butylène glycol | Eau | Butylène glycol | / | / |
| E | Ajout de fucogel à hauteur de 10% dans un mélange 50/50 (eau/ Butylène glycol) | Eau | Butylène glycol | 10 % Fucogel | / |
| F | Ajout de lutrol à hauteur de 10% dans un mélange 50/50 (eau/ Butylène glycol) | Eau | Butylène glycol | 10 % Lutrol | / |
| G | Extrait de mauve (10%mauve/45%eau/ 45% butylène glycol) | Eau | Butylène glycol | / | 10 % Mauve |
| H | Ajout de fucogel à hauteur de 10% dans l'extrait de mauve | Eau | Butylène glycol | 10 % Fucogel | 10 % Mauve |
| I | Ajout de lutrol à hauteur de 10% dans l'extrait de mauve | Eau | Butylène glycol | 10 % Lutrol | 10 % Mauve |

**Tableau 12**

| **Solution** | **Viscosité** | |
|---|---|---|
| A | 2,50 | mPa.s |
| B | 6,40 | mPa.s |
| C | 5,85 | mPa.s |
| D | 7,36 | mPa.s |
| E | 16,80 | mPa.s |
| F | 27,40 | mPa.s |
| G | 8,81 | mPa.s |
| H | 15,50 | mPa.s |
| I | 32,30 | mPa.s |

### Interprétation des résultats

La viscosité du mélange D (eau/butylène glycol dans les proportions 50/50) est de 7,36 mPa.s.

Lorsque l'on ajoute au mélange D du Fucogel (agent filmogène) à hauteur de 10% (mélange E), la viscosité est alors de 16,80 mPa.s. Ainsi, la contribution en viscosité apportée par le Fucogel est de l'ordre de 9,44 mPa.s (16,80-7,36).

Lorsque l'on ajoute au mélange D du Lutrol (agent viscosant) à hauteur de 10% (mélange F), la viscosité est alors de 27,40 mPa.s. Ainsi la contribution en viscosité apportée par le Lutrol est de l'ordre de 20,04 mPa.s (=27,40-7,36).

Lorsque l'on réalise la mesure de l'extrait de mauve (mélange G, composé de 10% mauve/45%eau/45%butylène glycol), la viscosité est alors de 8,81 mPa.s. Ainsi la contribution en viscosité apportée par la mauve est de l'ordre de 1,45 mPa.s (=8,81-7,36).

En réalisant le mélange H avec du fucogel à hauteur de 10%, de la mauve à hauteur de 10% dans le mélange de base eau/Butylène glycol, le résultat de viscosité attendue est de l'ordre de 18,25 mPa.s (=7,36+9,44+1,45).

Lors de l'essai, la mesure obtenue est de 15,50 mPa.s.

Ainsi, le résultat obtenu est inférieur à celui attendu. Cependant, comme déjà indiqué ci-dessus, la présence des plantes est connue pour diminuer le pouvoir viscosant des agents viscosants (et des agents gélifiants). Ici l'effet de la mauve abaisse la viscosité de la solution (15,50 mPa.s en présence de mauve pour le mélange H, contre 16,80 mPa.s pour la même solution sans mauve correspondant au mélange D).

En réalisant le mélange I avec du lutrol à hauteur de 10%, de la mauve à hauteur de 10% dans le mélange de base eau/Butylène glycol, le résultat de viscosité attendue est de l'ordre de 28,85 (=7,36+20,04+1,45). Lors de l'essai, la mesure obtenue est de 32,30 mPa.s.

Ainsi le résultat obtenu est supérieur à celui attendu. Ce résultat est d'autant plus surprenant puisque, comme indiqué précédemment, la présence de mauve aurait dû abaisser la viscosité.

### Conclusion

L'association de la mauve et du lutrol présente un effet inattendu sur la viscosité. Cette dernière se trouve amplifiée de près de 12% par rapport au résultat attendu.

### Exemple 8 : Etude de l'effet de la température sur la viscosité des compositions 1, 2 et 3 de l'invention

Les compositions de l'invention testées sont les compositions 1, 2 et 3 décrites respectivement dans les tableaux 1, 2 et 3 des exemples 1 et 2 ci-dessus.

Ces compositions sont comparées à un témoin ne comprenant pas d'émulsifiant non ionique à propriétés viscosifiantes.

La composition 13 du témoin est indiquée dans le tableau 13 ci-dessous :

**Tableau 13**

| Composition **11** (témoin) | **% (m/m)** |
|---|---|
| Extrait de Mauve (*Althea officinalis*) à 1-5% | 1% |
| Biosaccharide gum-1 à 1% (Fucogel® 1,5P) | 6% |
| Lutrol® F127 ou Lutrol® F68 | - |
| Sorbate de potassium | 0,125% |
| Acide citrique | Qsp pH=5 |
| Eau purifiée | Qsp 100% |

Différents essais ont été effectués avec le viscosimètre Rhéomat RM 200 dans les conditions suivantes :
- système de mesure 1.1 (godet 1 et mobile 1) ;
- précisaillement : 7 secondes à 500s⁻¹ ;
- cisaillement 15 secondes à 500 s⁻¹ ;
- 8 points de mesure ;
- température de 25°C.

Les valeurs de viscosités obtenues pour les compositions 11 (témoin), 1, 2 et 3 sont indiquées dans le tableau 14 ci-après :

**Tableau 14**

| | **Viscosités en mPa.s** | | | |
|---|---|---|---|---|
| **Températures** | **Compo.11 (témoin)** | **Compo. 1** | **Compo. 2** | **Compo. 3** |
| 15°C +/- 0,1°C | 5,35 | 7,32 | 12,30 | 7,73 |
| 20°C +/- 0,1°C | 4,97 | 6,18 | 10,40 | 6,41 |
| 25°C +/- 0,1°C | 4,63 | 5,88 | 8,87 | 5,87 |
| 30°C +/- 0,1°C | 4,30 | 5,57 | 7,60 | 5,76 |
| 35°C +/- 0,1°C | 3,74 | 5,16 | 6,16 | 5,38 |
| 40°C +/- 0,1°C | 2,98 | 4,15 | 5,09 | 4,45 |

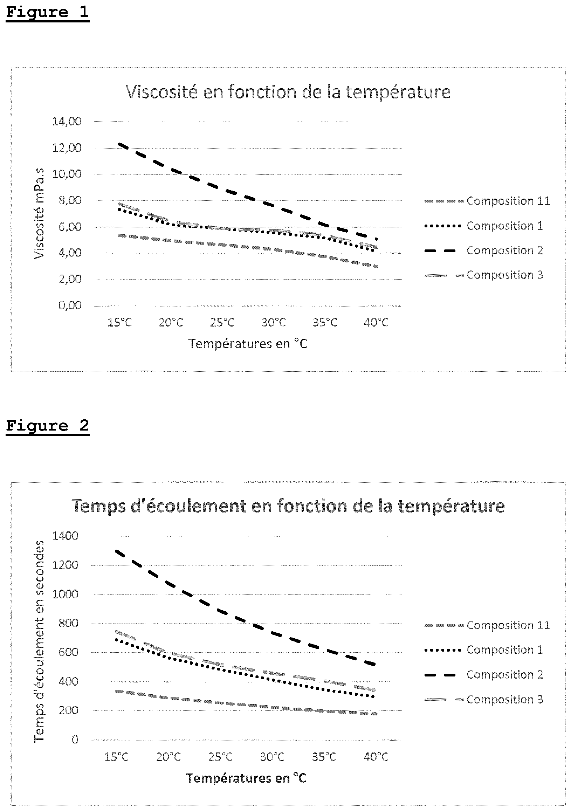

Les résultats du tableau 14 sont illustrés à la figure 1 (où la viscosité est représentée en fonction de la température).

### Interprétation des résultats

La composition 11 (témoin, ne comprenant pas de lutrol) a toujours les viscosités les plus faibles.

Les compositions 1 et 3 ont chacune 5% de Lutrol, (respectivement F68 et F127). Ces compositions ont un comportement et des viscosités très proches, elles évoluent de la même manière.

Ainsi, de 15°C à 25°C, leur viscosité va très légèrement diminuer, pour se stabiliser de 25°C à 35°C, puis, perdre un peu de viscosité (environ 1 mPa.s) à 40°C.

Quand la température augmente, la viscosité ne diminue que très légèrement.

Par contre, pour la composition 2, à 10% de de Lutrol (F68), la viscosité diminue lorsque la température augmente. Par contre les viscosités de la composition 2 sont plus élevées, pour une température donnée, que les viscosités des compositions 1 et 3.

L'augmentation de la quantité de Lutrol a donc un effet sur l'augmentation de la viscosité de la composition.

### Conclusion :

Le témoin (ne comprenant pas de lutrol) est plus liquide que les compositions 1 à 3 de l'invention et se liquéfie davantage quand la température augmente.

Quel que soit le type de Lutrol, celui-ci permet, à une concentration de 5%, d'obtenir une composition dont la viscosité ne variera que très peu sous une hausse de la température.

Par contre, pour une composition à 10% de Lutrol, la viscosité sera plus élevée que celle d'une composition à 5% de Lutrol, mais la viscosité diminuera plus lorsque la température augmente.

### Analyse du temps d'écoulement en fonction de la température

Les conditions opératoires sont celles utilisées pour mesurer une viscosité cinématique, à savoir un viscosimètre capillaire UBBELOHDE, numéro 1 (à savoir plage de viscosité allant de 2 à 10 mm².s⁻¹).

Les temps d'écoulement sont mesurés pour chaque composition à chaque température demandée.

### Mesure des temps d'écoulement

Les mesures réalisées sur chaque composition, par température, sont indiquées dans le tableau 15 ci-dessous :

**Tableau 15 :**

| | **Temps d'écoulement en secondes** | | | |
|---|---|---|---|---|
| **Températures** | **Compo. 11 (témoin)** | **Compo. 1** | **Campo. 2** | **Compo. 3** |
| 15°C | 335 | 687 | 1297 | 744 |
| 20°C | 288 | 563 | 1075 | 596 |
| 25°C | 254 | 485 | 887 | 517 |
| 30°C | 224 | 414 | 737 | 460 |
| 35°C | 198 | 345 | 620 | 407 |
| 40°C | 236 | 295 | 514 | 340 |

Les résultats du tableau 15 sont illustrés à la figure 2 (où le temps d'écoulement est représenté en fonction de la température).

### Interprétation des résultats

Comme observé lors des viscosités dynamiques mesurées au Rhéomat RM200, la composition témoin 11 présente les temps d'écoulement les plus faibles par rapport aux compositions 1 à 3 et à toutes les températures.

Par contre, contrairement aux mesures des viscosités dynamiques, les temps d'écoulement des compositions 1 et 3 diminuent toujours quand la température augmente, alors que les viscosités étaient plutôt assez stables quelle que soit la température.

### Conclusion :

Les compositions 11 (témoin) et 2 (10% Lutrol) ont leur temps d'écoulement qui évolue de la même façon que les viscosités dynamiques.

Ce n'est pas le cas des compositions 1 et 3.

Quelle que soit la méthode utilisée, on peut observer que l'absence de lutrol favorise la fluidification des compositions de mauve sous la hausse de la température.

Aussi, la présence du Lutrol, ainsi que sa concentration, aura une incidence sur le comportement des compositions de l'invention, à savoir leur viscosité et leur temps d'écoulement.

### Exemple 9 : test de fluorescéine sur une composition de l'invention

L'uniformité du film formé par la composition filmogène de l'invention, à savoir la composition 1 décrite dans le tableau 1, est observée à l'aide d'un test de fluorescéine.

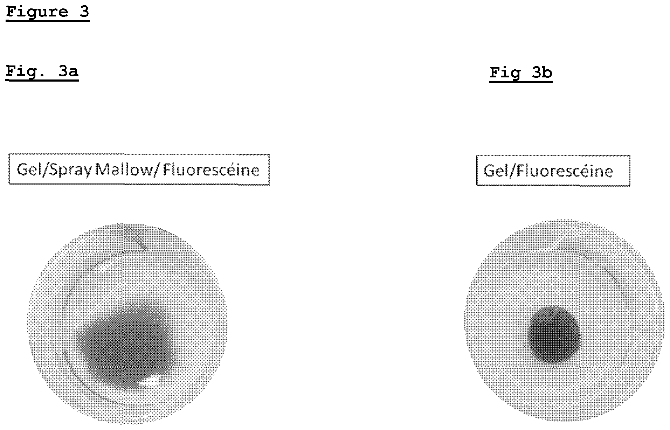

Les conditions du test sont les suivantes :
Utilisation de 8 mL d'un gel d'agar à 0,3% pour reproduire la muqueuse nasale. Dépôt dans un puits transparent. Température ambiante : 20°C.
Echantillon 1 (témoin) : dépôt de 50 µL d'une solution de fluorescéine par pipetage (voir figure 3b)
Echantillon 2 : Pulvérisation de la composition 1 de l'invention puis dépôt de 50 µL d'une solution de fluorescéine par pipetage (voir figure 3a).

On observe que la composition 1 de l'invention s'étale parfaitement bien (figure 3a), ce qui n'est pas le cas du témoin.

Les compositions de l'invention permettent donc d'obtenir un film uniforme.

Les mêmes résultats sont obtenus lorsque le test est effectué à une température de 37°C.

## Revendications

1. Composition filmogène, **caractérisée en ce qu'**elle comprend :
- un extrait de plantes de la famille des Malvaceae, de préférence du genre Althaea ou Malva,
- un agent filmogène, choisi dans le groupe comprenant un polysaccharide anionique ayant pour nom INCI biosaccharide gum-1, du xylitol, de l'acacia, du collagène, de l'acide hyaluronique et des mucopolysaccharides, et
- un émulsifiant non ionique, de préférence présentant des propriétés viscosifiantes, choisi dans le groupe comprenant un poloxamer, de la gomme xanthane, de la gomme de sclérotium et de l'agar agar, et est de préférence un poloxamer,
ladite composition étant exempte de chlorure de sodium.

2. Composition selon la revendication 1, **caractérisée en ce que** l'extrait de plantes est un extrait de Mauve, notamment choisi parmi *Malva sylvestris* ou Althaea *officinalis.*

3. Composition selon la revendication 1 ou la revendication 2, **caractérisée en ce que** l'agent filmogène est le polysaccharide anionique ayant pour nom INCI biosaccharide gum-1.

4. Composition selon l'une quelconque des revendications 1 à 3, **caractérisée en ce que** l'émulsifiant est un poloxamer choisi parmi le poloxamer 188 ou le poloxamer 407.

5. Composition selon l'une quelconque des revendications 1 à 4, **caractérisée en ce qu'**elle comprend en outre :
- un agent conservateur, de préférence choisi dans le groupe comprenant le sorbate de potassium, l'acide sorbique, l'éthylhexylglycérine, le pentylèneglycol, l'acide lactique et ses sels, et leurs mélanges,
- un agent ajusteur de pH, de préférence choisi dans le groupe comprenant l'acide citrique, l'acide acétique, l'acide chlorhydrique, et leurs mélanges,
- un solvant, de préférence choisi dans le groupe comprenant l'eau purifiée, l'eau osmosée, l'eau PPI, l'eau de mer naturelle ou reconstituée, l'eau permutée, le sérum physiologique, et leurs mélanges,
- éventuellement un actif, de préférence issu d'une plante habituellement utilisée pour la sphère ORL.

6. Composition selon la revendication 5, **caractérisée en ce que** :
- l'agent conservateur est le sorbate de potassium,
- l'agent ajusteur de pH est l'acide citrique,
- le solvant est l'eau purifiée,
- l'actif est une plante choisie dans le groupe comprenant l'eucalyptus, le thym, la marjolaine, l'origan, la camomille, le clou de girofle, la cannelle, le ravintsara, et leurs mélanges.

7. Composition selon l'une quelconque des revendications 1 à 6, dans laquelle:
- l'extrait de plantes, et notamment de fleurs de Mauve, est présent en une quantité en poids allant de 0,02% à 10%, de préférence de 0,05% à 1,5% en poids, et plus préférentiellement encore est présent en une quantité de 0,1% à 0,2%,
- l'agent filmogène, et notamment le polysaccharide anionique, est présent en une quantité en poids allant de 0,02% à 5,0%, de préférence de 0,04% à 0,8%, et plus préférentiellement encore est présent en une quantité de 0,06%,
- l'émulsifiant non ionique est présent en une quantité en poids allant de 0,01% à 15%, de préférence de 0,3% à 11%, et plus préférentiellement encore de 1% à 8%,
- l'agent conservateur est présent en une quantité allant de 0,07% à 0,5%, de préférence de 0,09% à 0,2%, et plus préférentiellement encore est présent en une quantité de 0,125% en poids,
- l'agent ajusteur de pH est ajouté en une quantité permettant un ajustement de pH allant de 2 à 8, de préférence de 3 à 7,5, et plus préférentiellement encore de 3,5 à 6,5,
- le solvant est ajouté en une quantité qsp 100%,
chacune desdites quantités en poids étant définie par rapport au poids total de la composition.

8. Utilisation non-thérapeutique d'une composition selon l'une quelconque des revendications 1 à 7, pour protéger les muqueuses notamment nasales, contre les agressions extérieures telles que les acariens, la pollution ou les pollens.

9. Composition selon l'une quelconque des revendications 1 à 7, pour une utilisation dans la prévention et/ou le traitement des symptômes de la rhinite allergique.

10. Composition pour une utilisation selon la revendication 9, pour une administration d'une ou deux pulvérisations dans chaque cavité nasale, deux à quatre fois par jour.

11. Dispositif de pulvérisation **caractérisé en ce qu'**il comprend une composition selon l'une quelconque des revendications 1 à 7, et **en ce qu'**il permet l'administration de ladite composition sur les muqueuses en doses.

12. Dispositif selon la revendication 11, **caractérisé en ce qu'**il se présente sous forme de flacon pompe, de flacon pressurisé en aérosol, de flacon pompe airless ou de valves à poches, et de préférence sous forme de flacon pompe.

13. Dispositif selon la revendication 11 ou 12 **caractérisé en ce qu'**il est adapté pour l'administration dans la cavité nasale.

## Patentansprüche

1. Filmbildende Zusammensetzung, **dadurch gekennzeichnet, dass** sie umfasst:
- ein Extrakt aus Pflanzen der Familie *Malvaceae,* vorzugsweise der Gattung *Althaea* oder *Malva,*
- ein filmbildendes Mittel, ausgewählt aus der Gruppe umfassend ein anionisches Polysaccharid mit dem Namen INCI biosaccharide gum-1, Xylitol, Akaziengummi, Kollagen, Hyaluronsäure und Mucopolysaccharide und
- ein nichtionisches Emulgiermittel, vorzugsweise mit viskositätserhöhenden Eigenschaften, das ausgewählt ist aus der Gruppe umfassend ein Poloxamer, Xanthangummi, Sklerotiumgummi und Agar-Agar und das vorzugsweise ein Poloxamer ist,
wobei die Zusammensetzung frei von Natriumchlorid ist.

2. Zusammensetzung gemäß Anspruch 1, **dadurch gekennzeichnet, dass** das Extrakt aus Pflanzen ein Extrakt aus Malven ist, insbesondere ausgewählt aus *Malva sylvestris* oder *Althaea officinalis.*

3. Zusammensetzung gemäß Anspruch 1 oder Anspruch 2, **dadurch gekennzeichnet, dass** das filmbildende Mittel das anionische Polysaccharid mit dem Namen INCI biosaccharide gum-1 ist.

4. Zusammensetzung gemäß einem der Ansprüche 1 bis 3, **dadurch gekennzeichnet, dass** das Emulgiermittel ein Poloxamer ist, das aus Poloxamer 188 oder Poloxamer 407 ausgewählt ist.

5. Zusammensetzung gemäß einem der Ansprüche 1 bis 4, **dadurch gekennzeichnet, dass** sie ferner umfasst:
- ein Konservierungsmittel, vorzugsweise ausgewählt aus der Gruppe umfassend Kaliumsorbat, Sorbinsäure, Ethylhexylglycerin, Pentylenglycol, Milchsäure und deren Salze und Mischungen davon,
- ein Mittel zum Einstellen des pH, vorzugsweise ausgewählt aus der Gruppe umfassend Zitronensäure, Essigsäure, Salzsäure und Mischungen davon,
- ein Lösungsmittel, vorzugsweise ausgewählt aus der Gruppe umfassend deionisiertes Wasser, Osmosewasser, Wasser für Injektionszwecke ("l'eau PPI"), natürliches oder rekonstituiertes Meerwasser, permutiertes Wasser, physiologische Kochsalzlösung und Mischungen davon,
- gegebenenfalls einen Wirkstoff, vorzugsweise aus einer Pflanze, die üblicherweise im Bereich der HNO verwendet wird.

6. Zusammensetzung gemäß Anspruch 5, **dadurch gekennzeichnet, dass**:
- das Konservierungsmittel Kaliumsorbat ist,
- das Mittel zum Einstellen des pH Zitronensäure ist,
- das Lösungsmittel deionisiertes Wasser ist,
- der Wirkstoff eine Pflanze ist, ausgewählt aus der Gruppe bestehend aus Eukalyptus, Thymian, Majoran, Oregano, Kamille, Nelke, Zimt, Ravintsara und Mischungen davon.

7. Zusammensetzung gemäß einem der Ansprüche 1 bis 6, wobei:
- das Extrakt aus Pflanzen, und insbesondere aus Malvenblüten, in einer Gewichtsmenge im Bereich von 0,02% bis 10%, vorzugsweise von 0,5 Gew.-% bis 1,5 Gew.-% und besonders bevorzugt in einer Menge von 0,1% bis 0,2% vorliegt,
- das filmbildende Mittel, und insbesondere das anionische Polysaccharid, in einer Gewichtsmenge im Bereich von 0,02% bis 5,0%, vorzugsweise von 0,04% bis 0,8% vorliegt und besonders bevorzugt in einer Menge von 0,06% vorliegt,
- das nichtionische Emulgiermittel in einer Gewichtsmenge im Bereich von 0,01% bis 15%, vorzugsweise von 0,3% bis 11%, und besonders bevorzugt von 1% bis 8%, vorliegt,
- das Konservierungsmittel in einer Menge im Bereich von 0,07% bis 0,5%, vorzugsweise von 0,09% bis 0,2% vorliegt und besonders bevorzugt in einer Menge von 0,125 Gew.-% vorliegt,
- das Mittel zum Einstellen des pH in einer Menge zugegeben ist, die es ermöglicht den pH im Bereich von 2 bis 8, vorzugsweise von 3 bis 7,5 und besonders bevorzugt von 3,5 bis 6,5 einzustellen,
- das Lösungsmittel in einer Menge von 100% Qs zugegeben ist, wobei jede der Gewichtsmengen in Bezug auf das Gesamtgewicht der Zusammensetzung definiert ist.

8. Nichttherapeutische Verwendung einer Zusammensetzung gemäß einem der Ansprüche 1 bis 7 zum Schutz der Schleimhäute, insbesondere der Nasenschleimhaut, vor aggressiven äußeren Einflüssen wie Milben, Umweltverschmutzung oder Pollen.

9. Zusammensetzung gemäß einem der Ansprüche 1 bis 7 zur Verwendung bei der Vorbeugung und/oder Behandlung der Symptome von allergischer Rhinitis.

10. Zusammensetzung zur Verwendung gemäß Anspruch 9 zur täglichen zwei- bis viermaligen Verabreichung von einem oder zwei Zerstäubungen in jede Nasenhöhle.

11. Sprühvorrichtung, **dadurch gekennzeichnet, dass** sie eine Zusammensetzung gemäß einem der Ansprüche 1 bis 7 umfasst und dass sie die dosisweise Verabreichung der Zusammensetzung an die Schleimhäute ermöglicht.

12. Vorrichtung gemäß Anspruch 11, **dadurch gekennzeichnet, dass** sie in Form einer Pumpflasche, einer Aerosoldruckflasche, einer luftlosen Pumpflasche ("flacon pompe airless") oder von Sackventilen und vorzugsweise in Form einer Pumpflasche vorliegt.

13. Vorrichtung gemäß Anspruch 11 oder 12, **dadurch gekennzeichnet, dass** sie zur Anwendung in der Nasenhöhle geeignet ist.

## Claims

1. Film-forming composition, **characterized in that** it comprises:
- an extract of plants of the family Malvaceae, preferably of the althaea or malva genus,
- a film-forming agent, chosen from the group comprising an anionic polysaccharide having the INCI name biosaccharide gum-1, xylitol, acacia, collagen, hyaluronic acid and mucopolysaccharides, and
- a non-ionic emulsifier, preferably having viscosifying properties, chosen from the group comprising a poloxamer, xanthan gum, sclerotium gum and agar agar, and which is preferably a poloxamer,
said composition being free of sodium chloride.

2. Composition according to Claim 1, **characterized in that** the extract of plants is an extract of mallow, in particular chosen from *Malva sylvestris* or *Althaea officinalis.*

3. Composition according to Claim 1 or Claim 2, **characterized in that** the film-forming agent is the anionic polysaccharide having the INCI name biosaccharide gum-1.

4. Composition according to any one of Claims 1 to 3, **characterized in that** the emulsifier is a poloxamer chosen from poloxamer 188 or poloxamer 407.

5. Composition according to any one of Claims 1 to 4, **characterized in that** it also comprises:
- a preservative, preferably chosen from the group comprising potassium sorbate, sorbic acid, ethylhexyl-glycerol, pentylene glycol, lactic acid and its salts, and mixtures thereof,
- a pH adjuster, preferably chosen from the group comprising citric acid, acetic acid, hydrochloric acid, and mixtures thereof,
- a solvent, preferably chosen from the group comprising purified water, osmosed water, water for injection, natural or reconstituted seawater, deionized water, physiological saline, and mixtures thereof,
- optionally an active agent, preferably derived from a plant normally used for the ENT sphere.

6. Composition according to Claim 5, **characterized in that**:
- the preservative is potassium sorbate,
- the pH adjuster is citric acid,
- the solvent is purified water,
- the active agent is a plant chosen from the group comprising eucalyptus, thyme, marjoram, oregano, camomile, clove, cinnamon, ravintsara, and mixtures thereof.

7. Composition according to any one of Claims 1 to 6, in which:
- the extract of plants, in particular of mallow flowers, is present in a weight amount ranging from 0.02% to 10%, preferably from 0.05% to 1.5% by weight, and even more preferentially is present in an amount of from 0.1% to 0.2%,
- the film-forming agent, and in particular the anionic polysaccharide, is present in a weight amount ranging from 0.02% to 5.0%, preferably from 0.04% to 0.8%, and even more preferentially is present in an amount of 0.06%,
- the non-ionic emulsifier is present in a weight amount ranging from 0.01% to 15%, preferably from 0.3% to 11%, and even more preferentially from 1% to 8%,
- the preservative is present in an amount ranging from 0.07% to 0.5%, preferably from 0.09% to 0.2%, and even more preferentially is present in an amount of 0.125% by weight,
- the pH adjuster is added in an amount which allows a pH adjustment ranging from 2 to 8, preferably from 3 to 7.5, and even more preferentially from 3.5 to 6.5,
- the solvent is added in an amount which is qs 100%,
each of said weight amounts being defined relative to the total weight of the composition.

8. Non-therapeutic use of a composition according to any one of Claims 1 to 7, for protecting the mucus membranes, in particular nasal mucus membranes, against external attacks such as acarids, pollution or pollen.

9. Composition according to any one of Claims 1 to 7, for use in the prevention and/or treatment of allergic rhinitis symptoms.

10. Composition for use according to Claim 9, for an administration of one or two sprays in each nasal cavity, two to four times a day.

11. Spray device **characterized in that** it comprises a composition according to any one of Claims 1 to 7, and **in that** it allows the mucosal administration of said composition in doses.

12. Device according to Claim 11, **characterized in that** it is in the form of a pump-dispenser bottle, a pressurized aerosol bottle, an airless pump-dispenser bottle or a bag on valve, and preferably in the form of a pump-dispenser bottle.

13. Device according to Claim 11 or 12, **characterized in that** it is suitable for administration in the nasal cavity.
